# EUROPEAN PATENT APPLICATION

(11) **EP 1 990 053 A1**
(43) Date of publication of application: **12.11.2008**
(21) Application number: 06834651.9
(22) Date of filing: 14.12.2006
(51) Int. Cl.: A61K 31/427, A61P 3/04, A61P 3/10, A61P 9/00, A61P 9/10, A61P 9/12, A61P 13/12, A61P 25/00, A61P 27/12, C07D 417/12

(54) **PHARMACEUTICAL COMPOSITION FOR ENHANCING ADIPONECTIN PRODUCTION**

(30) Priority: 16.12.2005 JP 2005362797
(71) Applicant: Daiichi Sankyo Company, Limited, Tokyo 103-8426 (JP)
(72) Inventor: KANDA, Shoichi, Shinagawa-ku, Tokyo 140-8710 (JP); OHSUMI, Jun, Shinagawa-ku Tokyo 140-8710 (JP)
(74) Representative: Gibson, Christian John Robert
(86) International application number: PCT/JP2006/324897
(87) International publication number: WO 2007/069669

(57) **Abstract**

[Object]

An object is to provide a compound having excellent adiponectin production enhancing action.

[Means for Solution]

A medicament comprising 5-[4-(6-methoxy-1-methyl-1H-benzimidazol-2-ylmethoxy)benzyl]thiazolidine-2,4-dione or a pharmacologically acceptable salt thereof as an active ingredient.

## Description

### [TECHNICAL FIELD]

The present invention relates to an adiponectin production enhancer which contains 5-[4-(6-methoxy-1-methyl-1H-benzimidazol-2-ylmethoxy)benzyl]thiazolidine-2,4-dione or a pharmacologically acceptable salt thereof as an active ingredient. In addition, the present invention relates to a therapeutic drug or a preventive drug for hypoadiponectinemia, Syndrome X or metabolic syndrome, and to a therapeutic drug or a preventive drug for diabetes, diabetic complications (including retinopathy, nephropathy, neuropathy, cataracts and coronary artery diseases), hypertension, obesity or arteriosclerosis caused by hypoadiponectinemia, which contains 5-[4-(6-methoxy-1-methyl-1H-benzimidazol-2-ylmethoxy)benzyl]thiazolidine-2,4-dione or a pharmacologically acceptable salt thereof as an active ingredient.

### [BACKGROUND ART]

Adiponectin is a protein that is specifically produced and secreted from adipocytes, and is intimately involved in energy balance and glucose or lipid metabolism (Maeda, K. et al., Biochemical and Biophysical Research Communications, 1996, Vol. 221, pp. 286-289). In actuality, in patients with circulatory diseases, diabetes or obesity, etc., blood adiponectin concentration decreases (Ouchi, N. et al., Circulation, 1999, Vol. 100, pp. 2473-2476; Lindsay, R.S. et al., Lancet, 2002, Vol. 360, pp. 57-58; Arita, Y. et al., Biochemical and Biophysical Research Communications, 1999, Vol. 257, pp. 79-83). In addition, kidney disease patients exhibiting low blood adiponectin concentrations are known to have a higher mortality rate due to circulatory diseases than patients with high blood adiponectin concentrations (Zoccali, C. et al., Journal of American Society of Nephrology, 2002, Vol. 13, pp. 134-141). Thus, disease states having decreased blood adiponectin concentrations, namely hypoadiponectinemia, are thought to be closely related to lifestyle diseases such as circulatory diseases (arteriosclerosis, hypertension, and the like), diabetes or obesity, and are believed to be one of their basic causes (Weyer, C. et al., The Journal of Clinical Endocrinology & Metabolism, 2001, Vol. 86, pp. 1930-1935; Hotta, K. et al., Diabetes, 2001, Vol. 50, pp. 1126-1133). Thus, the treatment or prevention of hypoadiponectinemia is also useful in the treatment or prevention of the aforementioned lifestyle diseases caused by hypoadiponectinemia.

Adiponectin is known to have actions of suppressing adhesion of THP-1 cells to vascular endothelial cells, expression of adhesion molecules, differentiation of vascular smooth muscle cells, macrophage foam cell formation, and the like (Ouchi, N. et al., Circulation, 1999, Vol. 100, pp. 2473-2476; Ouchi, N. et al., Circulation 2001, Vol. 103, pp. 1057-1063; Arita, Y. et al., Circulation 2002, Vol. 105, pp. 2893-2898; Ouchi, N. et al., Circulation, 2000, Vol. 102, pp. 1296-1301; Yokota, T. et al., Blood, 2000, Vol. 96, pp. 1723-1732). These phenomena in the living body are intrinsic phenomena that occur during the initial stage of the onset of arteriosclerosis (Ross, R. et al., Nature, 1993, Vol. 362, pp. 801-809), and the inhibitory effects demonstrated by adiponectin on these phenomena are extremely useful for the treatment or prevention of arteriosclerosis. In addition, increasing adiponectin concentration has been shown to have therapeutic effects on arteriosclerosis in an actual animal model (Okamoto, Y. et al., Circulation, 2002, Vol. 106, pp. 2767-2770).

In addition, adiponectin is also closely related to insulin resistance and diabetes (Kondo, H. et al., Diabetes, 2002, Vol. 51, pp. 2325-2328). Insulin resistance is known to increase in the presence of hypoadiponectinemia (Weyer, C. et al., The Journal of Clinical Endocrinology & Metabolism, 2001, Vol. 86, pp. 1930-1935; Hotta, K. et al., Diabetes, 2001, Vol. 50, pp. 1126-1133), and in an animal model, administration of adiponectin is known to demonstrate glucose metabolism ameliorative action by having effects of improving insulin resistance, suppressing glucose production in the liver, and the like (Yamauchi, T., et al., Nature Medicine, 2001, Vol. 7, pp. 941-946; Berg, A.H. et al., Nature Medicine, 2001, Vol. 7, pp. 947-953; Combs, T.P. et al., Clinical Investigation, 2001, Vol. 108, pp. 1875-1881). Thus, increasing blood adiponectin concentration is useful for the treatment or prevention of diabetes and diabetic complications caused thereby.

Disease states that exhibit increased insulin resistance, namely insulin resistance syndrome, are considered to be a principal cause of diabetes as well as the fundamental cause of lifestyle diseases such as circulatory diseases (arteriosclerosis, hypertension, and the like) or obesity (McVeigh, GE. et al., Current Diabetes Reports, 2003, Vol. 3, pp. 87-92; Chaudhuri, A. et al., Current Diabetes Reports, 2002, Vol. 2, pp. 305-310; Sorisky, A. et al., American Journal of Therapeutics, 2002, Vol. 9, pp. 516-521), and improvement of insulin resistance plays an important role in the treatment or prevention of the aforementioned lifestyle diseases. In other words, improvement of insulin resistance is also useful for the treatment or prevention of the aforementioned lifestyle diseases caused by insulin resistance syndrome. As previously mentioned, since adiponectin has an action that improves insulin resistance (Yamauchi, T. et al., Nature Medicine, 2001, Vol. 7, pp. 941-946), a drug that enhances adiponectin production is useful for the treatment or prevention of insulin resistance syndrome, as well as the treatment or prevention of diabetes, diabetic complications, circulatory diseases (arteriosclerosis, hypertension, and the like) and obesity caused by insulin resistance syndrome.

In addition, the concepts of Syndrome X, metabolic syndrome, and the like have recently been advocated as pathologies that increase the risk of coronary artery disease through a complex relationship of abnormal lipid metabolism diseases, diabetes and insulin resistance syndrome, and so forth (Reave, G.M., Diabetes, 1988, Vol. 37, pp. 1595-1607; DeFronzo, R.A. et al., Diabetes Cara, 1991, Vol. 14, pp. 173-194; Matsuzawa, Y, Nihon-Naikagaku-Zasshi, 1995, Vol. 84, pp. 209-212). As previously described, since adiponectin is able to contribute to the treatment or prevention of the respective causes of Syndrome X and metabolic syndrome, and the like, a drug that enhances the production of adiponectin is also useful for the treatment or prevention of Syndrome X and metabolic syndrome, and the like.

On the basis of the above, a drug that enhances adiponectin production possesses an action that improves insulin resistance, and is useful as an adiponectin production enhancer, a therapeutic drug or a preventive drug for hypoadiponectinemia, an insulin sensitiser, a therapeutic drug or a preventive drug for Syndrome X or metabolic syndrome, a therapeutic drug or a preventive drug for diabetes, diabetic complications (including retinopathy, nephropathy, neuropathy, cataract, and coronary artery disease), hypertension, obesity or arteriosclerosis, and as a therapeutic drug or a preventive drug for diabetes, diabetic complications (including retinopathy, nephropathy, neuropathy, cataracts and coronary artery disease), hypertension, obesity or arteriosclerosis that are caused by hypoadiponectinemia or insulin resistance syndrome.

Although certain types of thiazolidinedione compounds are known to demonstrate an action that enhances adiponectin production (for example, refer to non-Patent Document 1), 5-[4-(6-methoxy-1-methyl-1H-benzimidazol-2-ylmethoxy)benzyl]thiazolidine-2,4-dione or a pharmacologically acceptable salt thereof has not been known to demonstrate adiponectin production enhancing action or therapeutic or preventive effects for hypoadiponectinemia.
[non-Patent Document 1] Maeda, N. et al., Diabetes, 2001, Vol. 50, pp. 2094-2099.

### [DISCLOSURE OF THE INVENTION]

### [Problems to be Solved by the Invention]

The inventors of the present invention found that 5-[4-(6-methoxy-1-methyl-1H-benzimidazol-2-ylmethoxy)benzyl]thiazolidine-2,4-dione or a pharmacologically acceptable salt thereof has a superior adiponectin production enhancing action, and is useful as an adiponectin production enhancer, a therapeutic drug or a preventive drug for hypoadiponectinemia, a therapeutic drug or a preventive drug for Syndrome X or metabolic syndrome, and as a therapeutic drug or a preventive drug for diabetes, diabetic complications (including retinopathy, nephropathy, neuropathy, cataract and coronary artery disease), hypertension, obesity or arteriosclerosis that are caused by hypoadiponectinemia, thereby leading to completion of the present invention.

### [Means for Solving the Problems]

The present invention provides an adiponectin production enhancer, a therapeutic drug or a preventive drug for hypoadiponectinemia, a therapeutic drug or a preventive drug for Syndrome X or metabolic syndrome, a therapeutic drug or a preventive drug for diabetes, diabetic complications (including retinopathy, nephropathy, neuropathy, cataracts and coronary artery disease), hypertension, obesity or arteriosclerosis, and a therapeutic drug or a preventive drug for diabetes, diabetic complications (including retinopathy, nephropathy, neuropathy, cataract and coronary artery disease), hypertension or arteriosclerosis that are caused by hypoadiponectinemia, which contain 5-[4-(6-methoxy-1-methyl-1H-benzimidazol-2-ylmethoxy)benzyl]thiazolidine-2,4-dione or a pharmacologically acceptable salt thereof as an active ingredient.

The present invention is
(1) an adiponectin production enhancer which contains 5-[4-(6-methoxy-1-methyl-1H-benzimidazol-2-ylmethoxy)benzyl]thiazolidine-2,4-dione or a pharmacologically acceptable salt thereof as an active ingredient,
(2) an adiponectin production enhancer which contains 5-[4-(6-methoxy-1-methyl-1H-benzimidazol-2-ylmethoxy)benzyl]thiazolidine-2,4-dione hydrochloride as an active ingredient,
(3) a therapeutic drug or a preventive drug for hypoadiponectinemia which contains 5-[4-(6-methoxy-1-methyl-1H-benzimidazol-2-ylmethoxy)benzyl]thiazolidine-2,4-dione or a pharmacologically acceptable salt thereof as an active ingredient,
(4) a therapeutic drug or a preventive drug for hypoadiponectinemia which contains 5-[4-(6-methoxy-1-methyl-1H-benzimidazol-2-ylmethoxy)benzyl]thiazolidine-2,4-dione hydrochloride as an active ingredient,
(5) a therapeutic drug or a preventive drug for diabetes, diabetic complications, hypertension, obesity or arteriosclerosis caused by hypoadiponectinemia, which contains 5-[4-(6-methoxy-1-methyl-1H-benzimidazol-2-ylmethoxy)benzyl]thiazolidine-2,4-dione or a pharmacologically acceptable salt thereof as an active ingredient,
(6) a therapeutic drug or a preventive drug for diabetes, diabetic complications, hypertension, obesity or arteriosclerosis caused by hypoadiponectinemia, which contains 5-[4-(6-methoxy-1-methyl-1H-benzimidazol-2-ylmethoxy)benzyl]thiazolidine-2,4-dione hydrochloride as an active ingredient,
(7) use of 5-[4-(6-methoxy-1-methyl-1H-benzimidazol-2-ylmethoxy)benzyl]-thiazolidine-2,4-dione or a pharmacologically acceptable salt thereof for manufacturing an adiponectin production enhancer, and
(8) a method for the treatment of hypoadiponectinemia comprising administering a drug which contains 5-[4-(6-methoxy-1-methyl-1H-benzimidazol-2-ylmethoxy)benzyl]-thiazolidine-2,4-dione or a pharmacologically acceptable salt thereof as an active ingredient.

5-[4-(6-Methoxy-1-methyl-1H-benzimidazol-2-ylmethoxy)benzyl]thiazolidine-2,4-dione or a pharmacologically acceptable salt thereof can be produced easily in accordance with methods described in Japanese Patent Appication (Kokai) No. Hei 9-295970, EP Patent No. 0745600, US Patent No. 5,886,014 and pamphlet of International Publication No. WO 00/71540, or methods complying therewith.

5-[4-(6-Methoxy-1-methyl-1H-benzimidazol-2-ylmethoxy)benzyl]thiazolidine-2,4-dione or a pharmacologically acceptable salt thereof is a compound represented by the following structure: 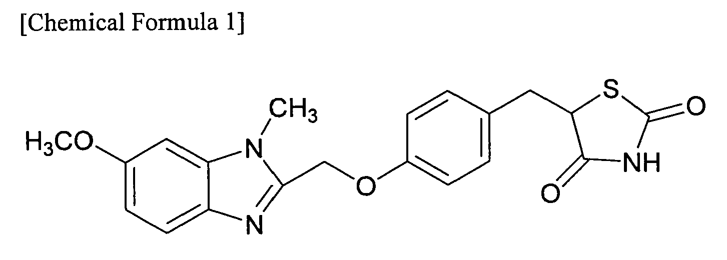 or a pharmacologically acceptable salt thereof, and is preferably 5-[4-(6-methoxy-1-methyl-1H-benzimidazol-2-ylmethoxy)benzyl]thiazolidine-2,4-dione hydrochloride.

The aforementioned 5-[4-(6-methoxy-1-methyl-1H-benzimidazol-2-ylmethoxy)benzyl]thiazolidine-2,4-dione, its racemic forms, its optical isomers and mixtures thereof are all embraced in the 5-[4-(6-methoxy-1-methyl-1H-benzimidazol-2-ylmethoxy)benzyl]thiazolidine-2,4-dione of the present invention. In addition, hydrates of the aforementioned 5-[4-(6-methoxy-1-methyl-1H-benzimidazol-2-ylmethoxy)benzyl]thiazolidine-2,4-dione or a pharmacologically acceptable salt thereof are also embraced in the 5-[4-(6-methoxy-1-methyl-1H-benzimidazol-2-ylmethoxy)benzyl]thiazolidine-2,4-dione or a pharmacologically acceptable salt thereof of the present invention.

5-[4-(6-Methoxy-1-methyl-1H-benzimidazol-2-ylmethoxy)benzyl]thiazolidine-2,4-dione or a pharmacologically acceptable salt thereof, which is an active ingredient compound of the present invention, can be used alone, and may also be used in combination with other drugs. In a case where two or more types of compounds are used, they can be used simultaneously, and may also be used separately at intervals of time.

In the case of using 5-[4-(6-methoxy-1-methyl-1H-benzimidazol-2-ylmethoxy)benzyl]thiazolidine-2,4-dione or a pharmacologically acceptable salt thereof, which is an active ingredient of the present invention, as a medicament (aforementioned therapeutic drug or preventive drug), it can be administered in the form of a bulk drug of the medicament itself, or it can be mixed with a suitable pharmacologically acceptable excipient, binder and the like to prepare a tablet, capsule, granules, pill, powder, liquid, syrup, troche, suspension or emulsion for oral administration, or an injection, suppository or patch, and the like for parenteral administration (preferably by oral administration).

These preparations are produced using well-known methods using additives such as excipients, binders, disintegration agents, lubricants, emulsifiers, stabilizers, correctives, diluents, injection solvents and the like.

An excipient may be, for example, an organic excipient or an inorganic excipient. Examples of organic excipients include sugar derivatives such as lactose, sucrose, glucose, mannitol and sorbitol; starch derivatives such as cornstarch, potato starch, alpha starch, dextrin and carboxymethyl starch; cellulose derivatives such as crystalline cellulose, hydroxypropyl cellulose, low substituted hydroxypropyl cellulose, hydroxypropylmethyl cellulose, carboxymethyl cellulose, calcium carboxymethyl cellulose, and internally-crosslinked sodium carboxymethyl cellulose; gum arabic; dextran; and pullulan. Examples of inorganic excipients include silicic acid salt derivatives such as light anhydrous silicic acid, synthetic aluminum silicate, calcium silicate, and magnesium metasilicate aluminate; phosphoric acid salts such as calcium phosphate; carbonic acid salts such as calcium carbonate; and sulfates such as calcium sulfate.

Examples of binders include the aforementioned excipient compounds; gelatin; polyvinylpyrrolidone; and polyethylene glycol.

Examples of disintegration agents include the aforementioned excipient compounds; chemically modified starch and cellulose derivatives such as crosscarmelose sodium and sodium carboxymethyl starch; and crosslinked polyvinylpyrrolidone.

Examples of lubricants include talc; stearic acid; metal stearates such as calcium stearate or magnesium stearate; colloidal silica; waxes such as beeswax and spermaceti; boric acid; glycol; DL-leucine; carboxylic acids such as fumaric acid and adipic acid; sodium carboxylate salts such as sodium benzoate; sulfates such as sodium sulfate; lauryl sulfates such as sodium lauryl sulfate and magnesium lauryl sulfate; silicic acids such as anhydrous silicic acid and silicic hydrate; and the above starch derivatives in the aforementioned excipients.

Examples of emulsifiers include colloidal clays such as bentonite and bee gum; metal hydroxides such as magnesium hydroxide and aluminum hydroxide; anionic surfactants such as sodium lauryl sulfate and calcium stearate; cationic surfactants such as benzalkonium chloride; and nonionic surfactants such as polyoxyethylene alkyl ether, polyoxyethylene sorbitan fatty acid ester, and sucrose fatty acid ester.

Examples of stabilizers include parahydroxybenzoates such as methyl paraben and propyl paraben; alcohols such as chlorobutanol, benzyl alcohol and phenylethyl alcohol; benzalkonium chloride; phenols such as phenol and cresol; thimerosal; anhydrous acetic acid; and sorbic acid.

Examples of correctives include ordinarily used sweeteners, sour flavorings and fragrances.

Examples of diluents include water, ethanol, propylene glycol, ethoxyisostearyl alcohol and polyoxyethylene sorbitan fatty acid esters.

Examples of injection solvents include water, ethanol and glycerin.

5-[4-(6-Methoxy-1-methyl-1H-benzimidazol-2-ylmethoxy)benzyl]thiazolidine-2,4-dione or a pharmacologically acceptable salt thereof, which is an active ingredient of the present invention, can be administered to a warm-blooded animal (particularly human). The dose can be varied depending on symptoms, age and the like of the patient. In the case of oral administration, 0.1 mg (preferably 0.5 mg, more preferably 1 mg) as a lower limit and 1000 mg (preferably 500 mg, more preferably 100 mg) as an upper limit, and in the case of parenteral administration, 0.01 mg (preferably 0.05 mg) as a lower limit and 100 mg (preferably 50 mg) as an upper limit can be administered per dosage, one or two times per day for adults depending on the symptoms.

The amount of 5-[4-(6-methoxy-1-methyl-1H-benzimidazol-2-ylmethoxy)-benzyl]thiazolidine-2,4-dione or a pharmacologically acceptable salt thereof which is contained in the aforementioned pharmaceutical preparation is not particularly limited and is selected from a wide range; however, it is generally appropriate that the amount contained is 1 to 70% by weight of the entire composition, preferably 1 to 30% by weight.

### [Effects of the Invention]

Since 5-[4-(6-methoxy-1-methyl-1H-benzimidazol-2-ylmethoxy)benzyl]-thiazolidine-2,4-dione or a pharmacologically acceptable salt thereof, which is an active ingredient of the present invention, has a superior adiponectin production enhancing action, it is useful as a therapeutic drug or a preventive drug for diseases in which blood adiponectin concentration decreases due to the occurrence of that disease, and diseases that occur due to a decrease in blood adiponectin concentration (and preferably diseases in which blood adiponectin concentration decreases due to the onset of that disease). The 5-[4-(6-methoxy-1-methyl-1H-benzimidazol-2-ylmethoxy)benzyl]thiazolidine-2,4-dione or a pharmacologically acceptable salt thereof, which is an active ingredient of the present invention, is useful as an adiponectin production enhancer, a therapeutic drug or a preventive drug for hypoadiponectinemia, a therapeutic drug or a preventive drug for Syndrome X or metabolic syndrome, and a therapeutic drug or a preventive drug for diabetes, diabetic complications (including retinopathy, nephropathy, neuropathy, cataracts and coronary artery disease), hypertension, obesity or arteriosclerosis that are caused by hypoadiponectinemia, preferably as an adiponectin production enhancer, a therapeutic drug or a preventive drug for hypoadiponectinemia, and a therapeutic drug or a preventive drug for diabetes, diabetic complications (including retinopathy, nephropathy, neuropathy, cataracts and coronary artery disease), hypertension, obesity or arteriosclerosis that are caused by hypoadiponectinemia, more preferably as an adiponectin production enhancer, a therapeutic drug or a preventive drug for hypoadiponectinemia, and a therapeutic drug or a preventive drug for diabetes or arteriosclerosis that are caused by hypoadiponectinemia, and even more preferably as an adiponectin production enhancer, and a therapeutic drug or a preventive drug for hypoadiponectinemia. In addition, the aforementioned therapeutic drug or preventive drug is preferred for warm-blooded animals, and more preferred for humans. The therapeutic drug or a preventive drug of the present invention is preferably a therapeutic drug.

### [BEST MODE FOR CARRYING OUT THE INVENTION]

The following provides a more detailed explanation of the present invention by indicating its examples and preparation examples, but the present invention is not limited thereto.

### [EXAMPLE]

5-[4-(6-Methoxy-1-methyl-1H-benzimidazol-2-ylmethoxy)benzyl]thiazolidine-2,4-dione hydrochloride (hereinafter referred to as Compound A), which is an insulin sensitizer, can be produced in accordance with methods described in Japanese Patent Appication (Kokai) No. Hei 9-295970, EP Patent No. 0745600, US Patent No. 5,886,014 and pamphlet of International Publication No. WO 00/71540. Rosiglitazone (hereinafter referred to as Compound B) can be produced in accordance with methods described in US Patent No. 5,002,953.

Pioglitazone (hereinafter referred to as Compound C) can be produced in accordance with methods described in US Patent No. 4,687,777.

### (Example 1)

### Comparative Test for Adiponectin Production Enhancing Effect of Compound A and Other Insulin Sensitizers

Female db/db mice of 6 weeks old (CLEA Japan, Inc.), which had developed diabetes, were divided into 4 groups of control group, Compound A administration group, Compound B administration group and Compound C administration group (5 mice/group).

All of the groups were free-fed with chow (FR-2, Funabashi Farms Co., Ltd.), and repeated oral administration was conducted for one week, with Compound A (0.3 mg/kg) for Compound A administration group, Compound B (3 mg/kg) for Compound B administration group, Compound C (30 mg/kg) for Compound C administration group, and administration vehicle only for control group, respectively. Plasma glucose levels and plasma adiponectin concentrations were compared between each of the groups respectively. The plasma adiponectin concentration was measured using a radioimmunoassay kit (Mouse Adiponectin RIA Kit, Linco Research, Inc.). Plasma was diluted by 100 times using a buffer provided with the kit, and measurement was conducted using the diluted plasma (5 µL) (final dilution rate: 2000 times). The radioactivity was measured using a gamma counter, and adiponectin concentration was calculated from the measured radioactivity (measurement unit: µg/mL). Results are shown in Table 1.

The adiponectin concentration one week after repeated administration increased significantly in each of the compound administration groups when compared with the control group. Compound A administration group showed significant increasing effect when compared with Compound B administration group and Compound C administration group respectively (Compound A administration group vs. Compound B administration group: P<0.001, Compound A administration group vs. Compound C administration group: P<0.01, based on Tukey's multiple comparison test). On the other hand, there was no significant difference between the Compound B administration group and the Compound C administration group, and no difference was observed among the three groups concerning the blood glucose levels.

From the above results, it can be said that 5-[4-(6-methoxy-1-methyl-1H-benzimidazol-2-ylmethoxy)benzyl]thiazolidine-2,4-dione or a pharmacologically acceptable salt thereof, which is an active ingredient of the present invention, possesses an especially superior adiponectin production enhancing effect compared with other insulin sensitizers, and is useful as an adiponectin production enhancer, a therapeutic drug or a preventive drug for hypoadiponectinemia, an insulin resistance improving drug, and as a therapeutic drug or a preventive drug for diabetes, diabetic complications (including retinopathy, nephropathy, neuropathy, cataracts and coronary artery disease), hypertension, obesity or arteriosclerosis that are caused by hypoadiponectinemia or insulin resistance syndrome.

### (Preparation Example 1)

### Capsules

| | |
|---|---|
| Compound A | 10 mg |
| Lactose | 95 mg |
| Corn starch | 58 mg |
| Magnesium stearate | 2 mg |
| | |
| Total | 165 mg |

Powders of each of the ingredients indicated above are thoroughly blended, and are passed through a sieve of 60 mesh (standard of mesh is in accordance with Tyler standard). 165 mg of the obtained powders are weighed out and are filled into a gelatin capsule to prepare a capsule.

### (Preparation Example 2)

### Tablets

| | |
|---|---|
| Compound A | 10 mg |
| Lactose | 95 mg |
| Corn starch | 34 mg |
| Microcrystalline cellulose | 25 mg |
| Magnesium stearate | 1 mg |
| | |
| Total | 165 mg |

Powders of each of the ingredients indicated above are thoroughly blended, and are compression molded into tablets, each of the tablets having 165 mg of weight. If necessary, these tablets may be coated with sugar or film.

### [INDUSTRIAL APPLICABILITY]

5-[4-(6-Methoxy-1-methyl-1H-benzimidazol-2-ylmethoxy)benzyl]thiazolidine-2,4-dione or a pharmacologically acceptable salt thereof, which is an active ingredient of the present invention, possesses superior adiponectin production enhancing action, and is thus useful as an adiponectin production enhancer, a therapeutic drug or a preventive drug for hypoadiponectinemia, a therapeutic drug or a preventive drug for Syndrome X or metabolic syndrome, and a therapeutic drug or a preventive drug for diabetes, diabetic complications (including retinopathy, nephropathy, neuropathy, cataracts and coronary artery disease), hypertension, obesity or arteriosclerosis that are caused by hypoadiponectinemia, preferably as an adiponectin production enhancer, a therapeutic drug or a preventive drug for hypoadiponectinemia, and a therapeutic drug or a preventive drug for diabetes, diabetic complications (including retinopathy, nephropathy, neuropathy, cataracts and coronary artery disease), hypertension, obesity or arteriosclerosis that are caused by hypoadiponectinemia, more preferably as an adiponectin production enhancer, a therapeutic drug or a preventive drug for hypoadiponectinemia, and a therapeutic drug or a preventive drug for diabetes or arteriosclerosis that are caused by hypoadiponectinemia, and even more preferably as an adiponectin production enhancer, and a therapeutic drug or a preventive drug for hypoadiponectinemia.

### [BRIEF DESCRIPTION OF THE DRAWINGS]

[FIG. 1]
   This is a diagram showing the plasma adiponectin concentration in a comparative test for adiponectin production enhancing action between Compound A and other insulin sensitizers (Example 1).

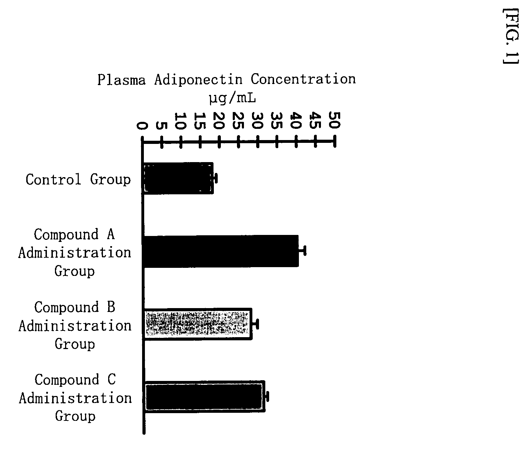

## Claims

1. An adiponection production enhancer comprising 5-[4-(6-methoxy-1-methyl-1H-benzimidazol-2-ylmethoxy)benzyl]thiazolidine-2,4-dione or a pharmacologically acceptable salt thereof as an active ingredient.

2. An adiponection production enhancer comprising 5-[4-(6-methoxy-1-methyl-1H-benzimidazol-2-ylmethoxy)benzyl]thiazolidine-2,4-dione hydrochloride as an active ingredient.

3. A therapeutic or preventive drug for hypoadiponectinemia comprising 5-[4-(6-methoxy-1-methyl-1H-benzimidazol-2-ylmethoxy)benzyl]thiazolidine-2,4-dione or a pharmacologically acceptable salt thereof as an active ingredient.

4. A therapeutic or preventive drug for hypoadiponectinemia comprising 5-[4-(6-methoxy-1-methyl-1H-benzimidazol-2-ylmethoxy)benzyl]thiazolidine-2,4-dione hydrochloride as an active ingredient.

5. A therapeutic drug or a preventive drug for diabetes, diabetic complications, hypertension, obesity or arteriosclerosis caused by hypoadiponectinemia comprising 5-[4-(6-methoxy-1-methyl-1H-benzimidazol-2-ylmethoxy)benzyl]thiazolidine-2,4-dione or a pharmacologically acceptable salt thereof as an active ingredient.

6. A therapeutic drug or a preventive drug for diabetes, diabetic complications, hypertension, obesity or arteriosclerosis caused by hypoadiponectinemia comprising 5-[4-(6-methoxy-1-methyl-1H-benzimidazol-2-ylmethoxy)benzyl]thiazolidine-2,4-dione hydrochloride as an active ingredient.

7. Use of 5-[4-(6-methoxy-1-methyl-1H-benzimidazol-2-ylmethoxy)benzyl]thiazolidine-2,4-dione or a pharmacologically acceptable salt thereof for manufacturing of an adiponectin production enhancer.

8. A method for the treatment of hypoadiponectinemia comprising administration of a drug comprising 5-[4-(6-methoxy-1-methyl-1H-benzimidazol-2-ylmethoxy)benzyl]thiazolidine-2,4-dione or a pharmacologically acceptable salt thereof as an active ingredient.
